# EUROPEAN PATENT SPECIFICATION

(11) **EP 4 401 700 B1**
(45) Date of publication and mention of the grant of the patent: **25.11.2026**
(21) Application number: 22773431.6
(22) Date of filing: 02.09.2022
(51) Int. Cl.: A61K 8/03, A61K 8/31, A61K 8/37, A61K 8/41, A61K 8/44, A61Q 1/00, A61Q 1/10

(54) **BI-PHASE MAKE-UP REMOVER**
ZWEIPHASIGER ABSCHMINKENTFERNER
DÉMAQUILLANT BIPHASÉ

(30) Priority: 16.09.2021 WO PCT/CN2021/118661
(43) Date of publication of application: 24.07.2024
(73) Proprietor: Beiersdorf AG, 22529 Hamburg (DE)
(72) Inventor: LI, Na, Wuhan, Hubei 430000 (CN); WANG, Ziyin, Wuhan, Hubei 430000 (CN)
(74) Representative: Beiersdorf AG
(86) International application number: PCT/EP2022/074464
(87) International publication number: WO 2023/041349

(56) References cited:
- EP-A1- 0 603 080
- EP-A1- 2 732 805
- US-A1- 2007 020 214
- DATABASE GNPD [online] Mintel; 2 September 2019 (2019-09-02), CELLTRION SKINCURE: "Glow Essential Mist", XP093008152, Database accession no. 6822505

## Description

### Technical field

The present invention belongs to the cosmetic field, and relates to a make-up remover composition, especially an eye make-up remover composition. The composition has an effective cleansing performance and at the same time does not leave the skin with a greasy or oily feeling after usage. The composition is made available in form of bi-phase make-up remover.

### Background art

Generally, make-up is used to improve the outward appearance, in particular the appearance of the face. Already in ancient times make-up was used. In Egypt, at the times when the pharaohs were governing the country, the use of make-up for the eyes, lips and cheeks was common. Women and men used it to protect the skin against the sun, but also for beautification. Nowadays, make-up is merely used to improve in particular the outward appearance of the face.

The applied make-up has to be removed. This may be achieved by face cleansers or products solely developed to remove make-up, so called make-up removers. The skin of the face is a relatively sensitive skin; therefore, it is recommended to use mild cleansing compositions.

Make-up compositions contain a great number of raw materials, which are of very different chemical nature. The dyes, contained in make-up compositions, are selected from inorganic pigments such as silicates (magnesium silicate (talc), aluminum silicate (kaolin)), and metal oxides (chrome, iron, manganese, titanium, and zinc oxides), and/or organic pigments. Binding means may be esters of stearic acid, lanolin alcohol and lanolin acetate. Furthermore, waxes, such as for instance, beeswax or carnauba wax, and oils, such as for instance, paraffin oil, silicone oil or castor oil, may be contained. Additionally, preservatives, antioxidants, thickeners, and other additives may be comprised.

To achieve a proper face cleansing, these different compounds have to be removed from the skin. At the same time nonpolar substances such as waxes, oils and silicone oils and pigments of low solubility, such as talc or titanium oxide have must be solubilized. This holds true for foundations, lipsticks, eye shadows, eye pencils, and also for long-lasting and waterproof products.

Prior art discloses face cleansing compositions suitable to remove make-up either. Different types of compositions are described, and respective products are in the market. The compositions may be in form of emulsions, creamy, milky, or liquid ones, gels, and oily compositions.

Compositions intended to remove make-up, especially eye make-up may be provided as bi-phase products, in general, having two distinct phases. Upon shaking a homogenous emulsion is formed, which is distributed on a pad or tissue or the hands and applied to the area around the eyes to remove make-up and dirt. Afterwards, again at rest, the phases will separate again. However, this dephasing process should not take place too rapidly. The user shall apply a homogenous mixture of both phases to the skin to take advantage of these both phases.

This special form of an eye make-up removing composition is known in prior art.

In the document EP 0603080 B1 a bi-phase cosmetic composition is described which contains in an oily phase mineral, vegetable and/or synthetic oils, including silicone oils, and in the aqueous phase a surfactant, preferably a nonionic surfactant and a dephasing agent, namely benzalkonium chloride.

A further dephasing agent, namely sodium bicarbonate, is disclosed in the document EP 1514534 A1, in which a biphasic make-up remover is described, too.

In the document EP 1894994 A1 two- or multi-phase compositions are disclosed, intended to remove make-up, especially eye make-up. All the examples represent compositions containing a fluid silicone oil, especially a cyclomethicone.

In the documents EP 2732805 A1 and EP 3270877 B1 a further dephasing agent, namely Benzethonium Chloride is disclosed. Compositions free from silicones are described in EP 3270877 B1.

The document US 10,537,502 B2 discloses a two-phase make-up removal composition comprising an aqueous phase and a separate water-in-oil or oil-in-water emulsion wherein both phases are clear.

Consumers do like biphasic compositions for the removal of make-up, especially eye make-up. However, a greasy and/or oily feeling of the skin after using such products is a frequent complaint of the consumers.

Furthermore, biphasic compositions need to be mild, because the skin of the face is quite sensible, which holds true even more for the skin of the area around the eyes. The challenging problem is to develop a mild, non-greasy make-up remover, which has the good cleansing performance to really remove the make-up and the dirt.

In addition, the consumer likes new products with a different or surprising appearance in comparison to known products. Bi-phase make-up remover having two clear phases are already on the market, as disclosed in the Mintel data base; for example, a Double Effect Eye Make-Up remover having the Mintel number 8772181 or an Eye Make-Up Remover Care having the Mintel number 8034659.

Further, the Mintel GNPD Record ID 6822505 discloses a skin care composition having two visually distinct phases. The document EP 2732805 A1 discloses that benzethonium chloride can be used for slowing down the phase separation in bi-phase cosmetic compositions. Further, EP 603080 A1 discloses benzethonium chloride as dephasing agent. Additionally, US 2007020214 A1 discloses the use of acylglutamic acid surfactants in dual or multiple phase systems.

In order to introduce a different appearance, the idea of a bi-phase make-up remover having a milky upper phase and a clear bottom phase came up.

### Summary of the invention

Hence the problem of the present invention can be summarized to provide a bi-phase make-up remover, being a mild but effectively cleansing one without leaving a greasy feeling on the skin. Moreover, a different appearance would be desirable.

Surprisingly, these requirements were met by a bi-phase make-up remover composition having two distinct phases at rest, wherein the upper phase is an emulsified oily phase with a milky appearance, and upon shaking a visibly homogeneous emulsion is formed, which is distributed onto the skin.

Hence, the bi-phase make-up remover composition, especially an eye make-up remover composition, contains
- the emulsifier sodium stearoyl glutamate,
- a dephasing agent, which is Benzethonium Chloride, and
- an oily phase comprising a least one iso-alkane, at least one alkane and at least one ester oil.

The composition according to the present invention is a cosmetic composition intended to be applied to the human skin, especially the face, even more especially the area around the eyes (eye make-up remover).

The composition according to the present invention is a bi-phase composition having an upper phase, which is milky, and a bottom phase, which is transparent. The phase boundary is clearly visible. The upper phase is an emulsified oily phase. The milky appearance of the upper phase is due to the emulsification.

Upon shaking a visibly homogenous emulsion is formed, which may be distributed on a pad or tissue or the hands and then applied to the face skin and/or the area around the eyes to remove make-up and dirt.

The weight ratio of the oily phase to an aqueous phase (a mixture of the emulsifier, the dephasing agent and optionally further substances in water) is from 10 : 90 to 50 : 50.

The composition according to the present invention contains an emulsifier, namely sodium stearoyl glutamate, the structure of which may be illustrated by the following graph:

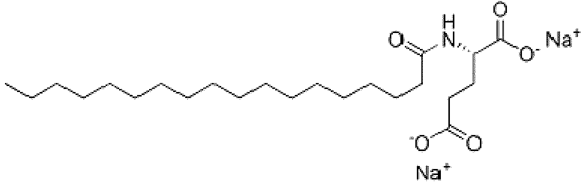

For example, the emulsifier sodium stearoyl glutamate may be purchased as Emulgin SG from the company of BASF.

Advantageously, the emulsifier sodium stearoyl glutamate may be contained in an amount of 0.05 to 0.5 % by weight, in relation to the active content and in relation to the total weight of the composition.

According to the present invention one or more additional emulsifier(s) may be contained, which may be selected from PEG-40 Hydrogenated Castor Oil and/or Glyceryl Stearate Citrate.

In order to avoid a greasy feeling of the skin after use of a make-up remover, the upper, oily phase is emulsified. The phase distribution of an emulsified upper phase and a clear (transparent) bottom phase results in a satisfying cleansing performance without any oily or greasy feeling of the skin after usage.

According to the present invention at least one iso-alkane is contained. Iso-alkanes, for which sometimes the expression iso-paraffins is used, comprise aliphatic, branched hydrocarbons having in total 12 to 40 carbon atoms, preferably 12 to 16 carbon atoms. It is preferred, if isohexadecane and/or isododecane are contained in the composition according to the present invention.

Advantageously, in the composition according to the present invention the at least one iso-alkane is contained in a total amount of 1.0 to 90 % by weight, preferably 5.0 to 40 % by weight, in relation to the total weight of the composition.

According to the present invention at least one alkane is contained. Alkanes, for which sometimes the expression paraffins is used, comprise aliphatic, unbranched hydrocarbons having 12 to 40 carbon atoms, preferably 15 to 19 carbon atoms. Said hydrocarbons may be contained as components being characterized by one specific number of carbon atoms. However, it is preferred, if blends of hydrocarbons having 15 to 19 carbon atoms (e.g. C15-19 alkane) are contained. For example, such a blend may be purchased from Total as Gemseal 40.

Advantageously, in the composition according to the present invention the at least one alkane is contained in a total amount of 1.0 to 15 % by weight, preferably 1.2 to 7.5 % by weight, in relation to the total weight of the composition.

According to the present invention at least one ester oil is contained. Ester oils are esters of saturated and/or unsaturated, branched and/or unbranched alkane carboxylic acids with a chain length of from 3 to 20 carbon atoms and saturated and/or unsaturated, branched and/or unbranched alcohols with a chain length of from 1 to 20 carbon atoms, and esters of aromatic carboxylic acids and saturated and/or unsaturated, branched and/or unbranched alcohols with a chain length of from 3 to 20 carbon atoms, with the proviso that said esters are fluid oils. Examples for such ester oils are isopropyl myristate, isopropyl palmitate, coco caprylate/caprate, and/or C12-15 -alkyl benzoate.

Advantageously, in the composition according to the present invention the at least one ester oil is contained in a total amount of 0.05 to 10 % by weight, preferably 0.1 to 3.0 % by weight, in relation to the total weight of the composition.

According to the present invention the composition is free from silicone components. Silicones are synthetic components, mostly in form of polymers, containing silicium atoms connected via oxygen atoms. The functional unit mainly consisting of silicium atom and oxygen atom is called siloxane, and has the general formula: RₙSiO_{(4-n)/2} (n=0, 1, 2, 3, R= chemical substituents, commonly methyl group).

The term "free from" is intended to mean a composition containing less than 0.1 % by weight, preferably less than 0.01 % by weight, more preferably 0.001 % by weight, most preferably 0 % by weight, of a respective component, relative to the total weight of the composition.

Make-up removal biphasic systems have been shown to perform more effectively than compositions only having an oily or an aqueous phase. The solubilization capacity of oils and aqueous cleansing components is combined.

To form a two-phase product, an aqueous and an oily phase are required. In the resting state, these two phases are ideally optically clearly separated from each other. Due to the different densities, these two phases do not mix independently and thus form a horizontally separated two-phase system. By introducing energy, e.g. by shaking, the phases can be mixed together for a short time. However, due to the difference in density and a high interfacial tension (IFT) of oil compared to water, afterwards a very rapid separation into the two phases occurs.

In order to enable the use of two-phase products, the separation speed and in particular the separation start should advantageously be slowed down, since otherwise only one phase, or mostly only one phase, is distributed from the packaging and used. The consequence would be a lower cleansing performance because the cleansing effect would be reduced due to the missing phase.

By adding Benzethonium Chloride to the bi-phase composition, a prolongation of the start of the phase separation may be achieved after having mixed the two phases.

The separation start is the time at which the separation of the two phases becomes visually apparent and completely separated phases are not yet present. The separation time can be extended by the addition of Benzethonium Chloride, usually by 20 to 25 seconds, in individual cases up to 15 minutes. This effect allows for an effective usage of bi-phase compositions as the time until the phases are completely separated is sufficiently delayed.

The separation into two phases with a clear interface is usually approximately complete after about 15 minutes; the phases may then still be slightly turbid, depending on how vigorously and for how long they have been shaken. After < 12 hours both phases are distinctly separated again.

In addition to the effect described above, Benzethonium Chloride has an antimicrobial effect.

In the composition according to the present invention Benzethonium Chloride is contained in an amount of 0.01 to 0.1 % by weight, preferably 0.03 to 0.06 % by weight, in relation to the total weight of the composition.

Additionally, the composition according to the invention may contain at least one nonionic surfactant, especially at least one alkyl poly glucoside.

Alkyl poly glucosides may be represented by the formula RO-(Z)ₓ, wherein R is the alkyl group having 6 to 30 carbon atoms, Z is a sugar moiety and x is the number of sugar moieties. The alkyl poly glucosides according to the invention may have a specific alkyl group, representing one distinct number of carbon atoms. In general, alkyl poly glucosides are produced starting from natural oils, preferably plant oils, more preferably coconut oil. In this case, the alkyl group R is a mixture of the alkyl groups of the respective fatty acids of natural oil used as a starting material.

It is preferred, if the alkyl poly glucosides have alkyl groups, where R essentially consists of C8 to C18 alkyl groups, preferably C8 to C12 alkyl groups.

The alkyl poly glucosides contain 1.1 to 5 sugar moieties on average, preferably 1.1 to 2.0, more preferably 1.1 to 1.8. It is most preferred if the alkyl poly glucoside is caprylyl/capryl glucoside. Caprylyl/capryl glucoside may be purchased from BASF as PLANTACARE 818 UP.

Advantageously, in the composition according to the invention the at least one alkyl poly glucoside is contained in an amount of 0.05 to 0.5 % by weight, preferably 0.1 to 0.3 % by weight, in relation to the active content and in relation to the total weight of the composition.

Advantageously, according to the invention additionally at least one electrolyte may be contained in the composition of the present invention. Electrolytes may be termed mineral nutrients. According to the present invention the electrolytes may be chosen from potassium, magnesium, calcium, sodium, chloride, and/or phosphate. Electrolytes may be absorbed by the skin. Due to their charge they are hydrated and hence, may influence the hydration of the skin in a positive manner. The at least one electrolyte may be sodium chloride.

If at least one electrolytes is contained in the composition of the present invention, the at least one electrolytes is contained in an amount of 0.1 to 2 % by weight, preferably 0.2 to 0.7 % by weight, in relation to the total weight of the composition.

Advantageously, according to the invention additionally at least one moisturizer is contained in the composition of the present invention. Moisturizers are hygroscopic substances which bind water and thereby provide moisture. The hygroscopic quality is due to hydrophilic substituents of the molecule, in many cases hydroxyl groups, but other functional groups for instance, amine or carboxyl groups may act in the same way. Examples of moisturizers are propylene glycol, hexylene glycol, and butylene glycol, glycerol, sorbitol, xylitol, maltitol, and/or glyceryl glucoside. Preferred moisturizers are glycerol, butylene glycol and/or maltitol.

If at least one moisturizer is contained in the composition of the present invention, the at least one moisturizer is contained in a total amount of 1.0 to 10 % by weight, preferably 1.2 to 5 % by weight, in relation to the total weight of the composition.

Chelating agents are well-known additives in the cosmetic field. By complexing metals like Mn, Fe, Cu, and others, undesirable chemical reactions may be prevented.

Advantageously, the chelating agent or agents may be selected from the group of aminopolycarboxylic acids and its anions (such as ethylene diamine tetraacetic acid (EDTA) and its anions, nitrilotriacetic acid (NTA) and its anions, hydroxyethylenediaminotriacetic acid (HOEDTA) and its anions, diethylenaminopentaacetic acid (DPTA) and its anions, trans-1,2-diaminocyclohexanetetraacetic acid (CDTA) and its anions) and tetrasodium iminodisuccinate.

If at least one chelating agent is contained in the composition of the present invention, the at least one chelating agent is contained in a total amount of 0.01 to 5.0 % by weight, preferably 0.6 to 1.5 % by weight, in relation to the total weight of the composition.

Advantageously, the composition according to the present invention contains preservatives. All preservatives, which are allowed and suitable for cosmetic compositions may be contained in the composition according to the invention.

It is preferred, if Phenoxyethanol is contained in the composition according to the invention. Phenoxyethanol is contained in an amount of 0.01 to 1.2 % by weight, preferably 0.05 to 1.0 % by weight, more preferably 0.1 to 0.9 % by weight in the composition according to the invention, in relation to the total weight of the composition.

The present invention also relates to a process for the preparation of the composition according to any one of the preceding claims comprising the following steps:
- Dissolve the emulsifier sodium stearoyl glutamate in water,
- add Benzethonium Chloride and optionally further substances to this mixture (aqueous phase),
- prepare the oily phase by mixing the iso-alkane(s), alkane(s), and ester oil(s),
- mix the oily phase and aqueous phase in a beaker,
- homogenize the mixture at 3 to 8 K RPM for 20 to 360s.

### Brief description of the Drawings

Fig.1 shows evaluation of different emulsifiers for compositions after preparation (Fig.1A) and compositions after shaking for six to ten seconds and a rest for 12 hours (Fig.1B), wherein * % by weight referring to the aqueous phase;
Fig. 2 shows results of different weight ratios of the oily phase to the aqueous phase (O/W ratio) for compositions after shaking for six to ten seconds and a rest for 12 hours;

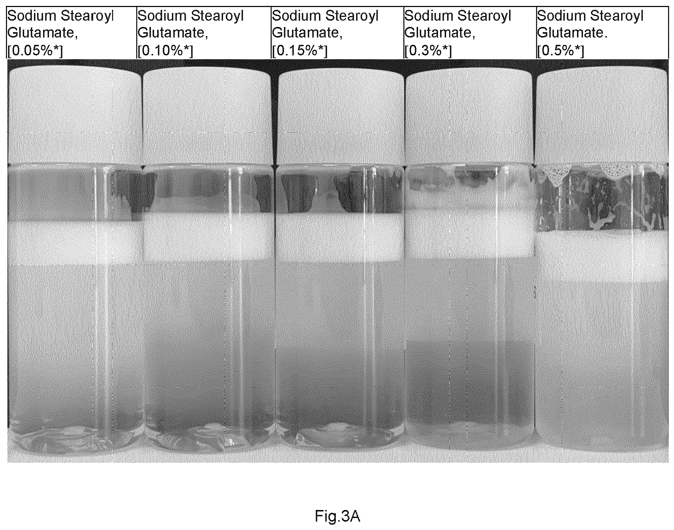

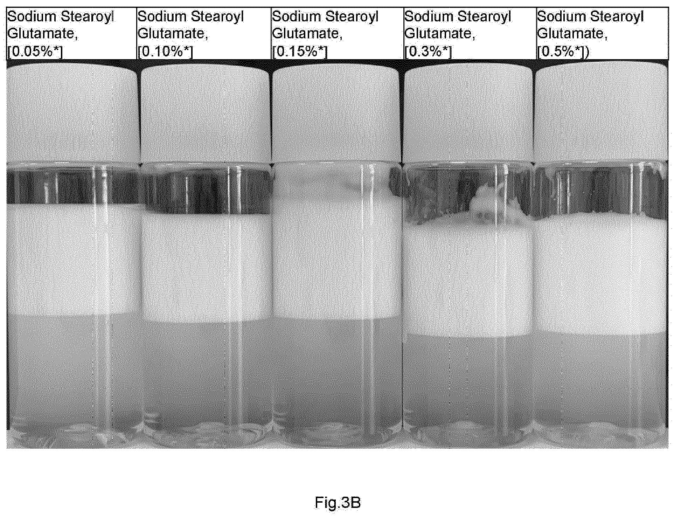

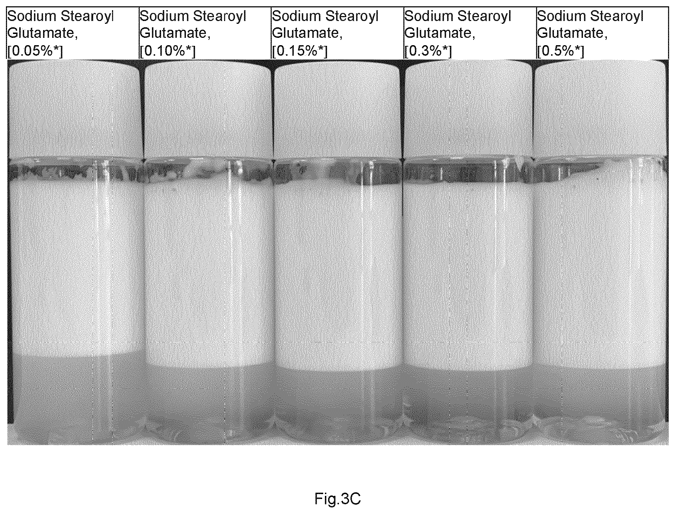

Fig.3 shows evaluation of impacts of different weight ratios of the oily phase to the aqueous phase and different concentrations of the emulsifier on the phase separation after shaking for six to ten seconds and a rest for 12 hours, wherein Fig.3A shows weight ratio of the oily phase to the aqueous phase 10 : 90 (table 3, examples 1 - 5), Fig.3B shows weight ratio of the oily phase to the aqueous phase 30 : 70 (table 3, examples 6 - 10), and Fig.3C shows weight ratio of the oily phase to the aqueous phase 50 : 50 (table 3, examples 11 - 15), and wherein * % by weight referring to the aqueous phase; and

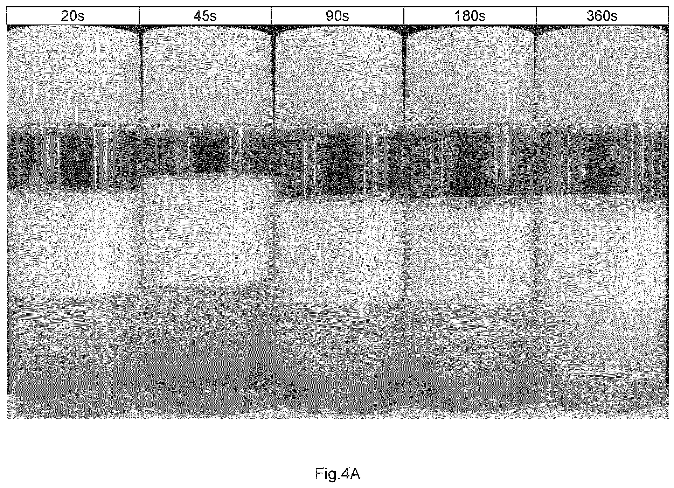

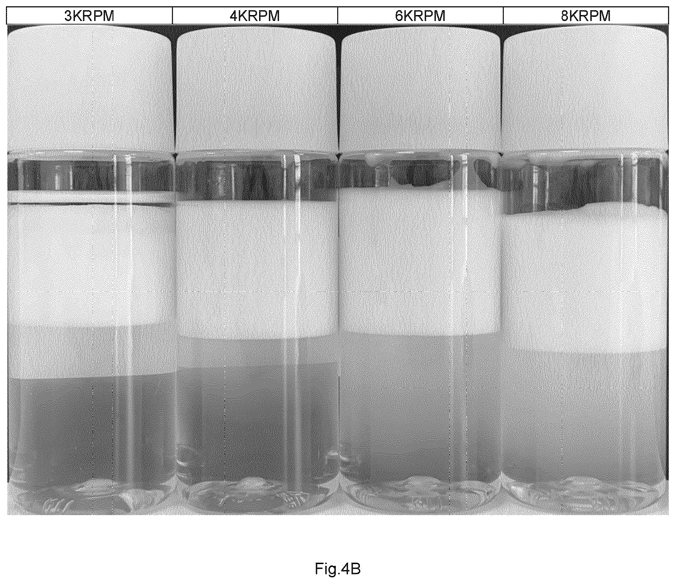

Fig.4 shows evaluation of the conditions of preparation, wherein Fig.4A shows duration of homogenization, and Fig.4B shows speed of homogenization, for compositions after shaking for six to ten seconds and a rest for 12 hours.

### Examples

The examples are intended to illustrate the present invention without limiting it. The numerical values in the examples are percentages by weight, based on the total weight of the particular preparations. The values are referring to active content, otherwise two asterisks (**) indicate the use of a solution.

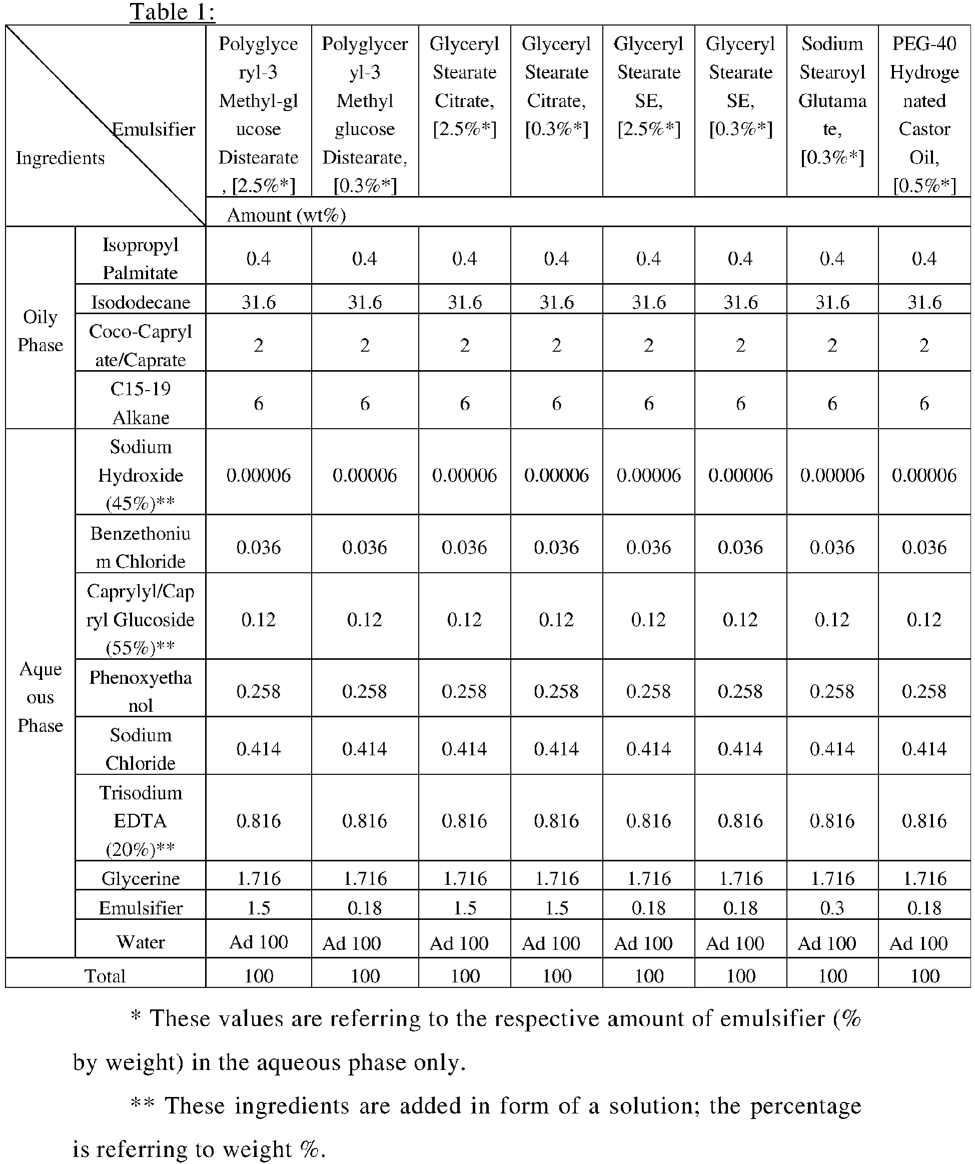

### Sample Preparation:

1) Weigh the emulsifier according to the amounts shown in the table above and dissolve in water.
2) Use the emulsifier containing water to prepare the aqueous phase as shown in the table above.
3) Prepare the oily phase according to the table above.
4) Mix the oily phase and aqueous phase in a beaker.
5) Homogenize the mixture 45 seconds at 8K RPM.
6) Transfer the compositions into sample bottles.

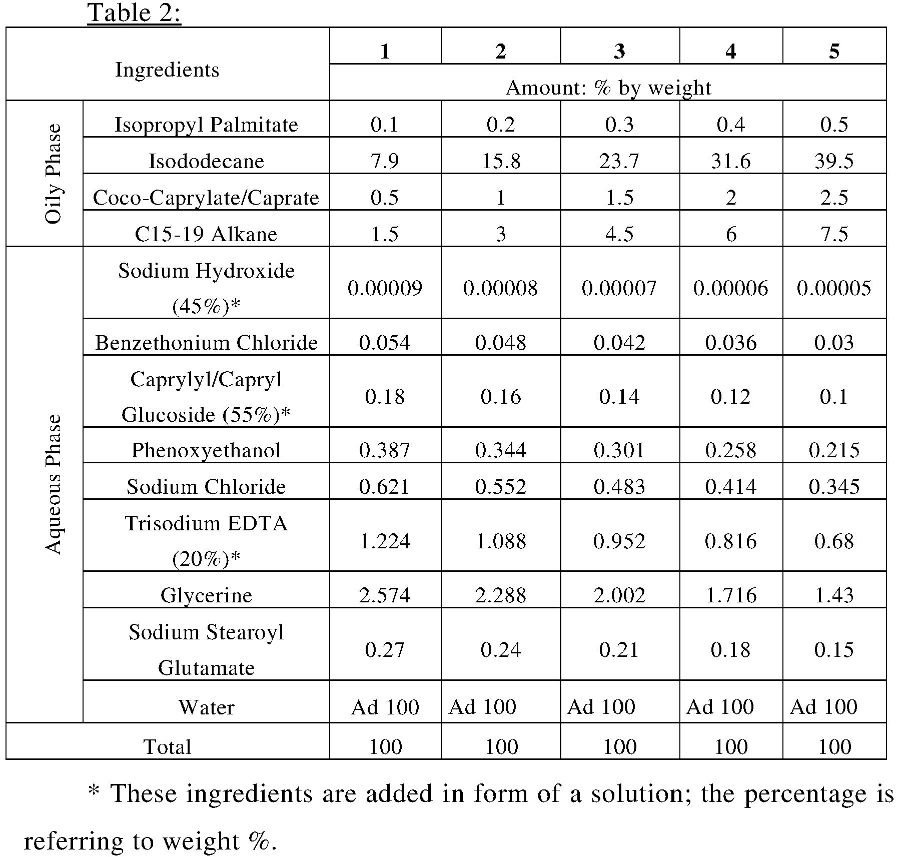

Sample Preparation: The same as indicated for table 1.

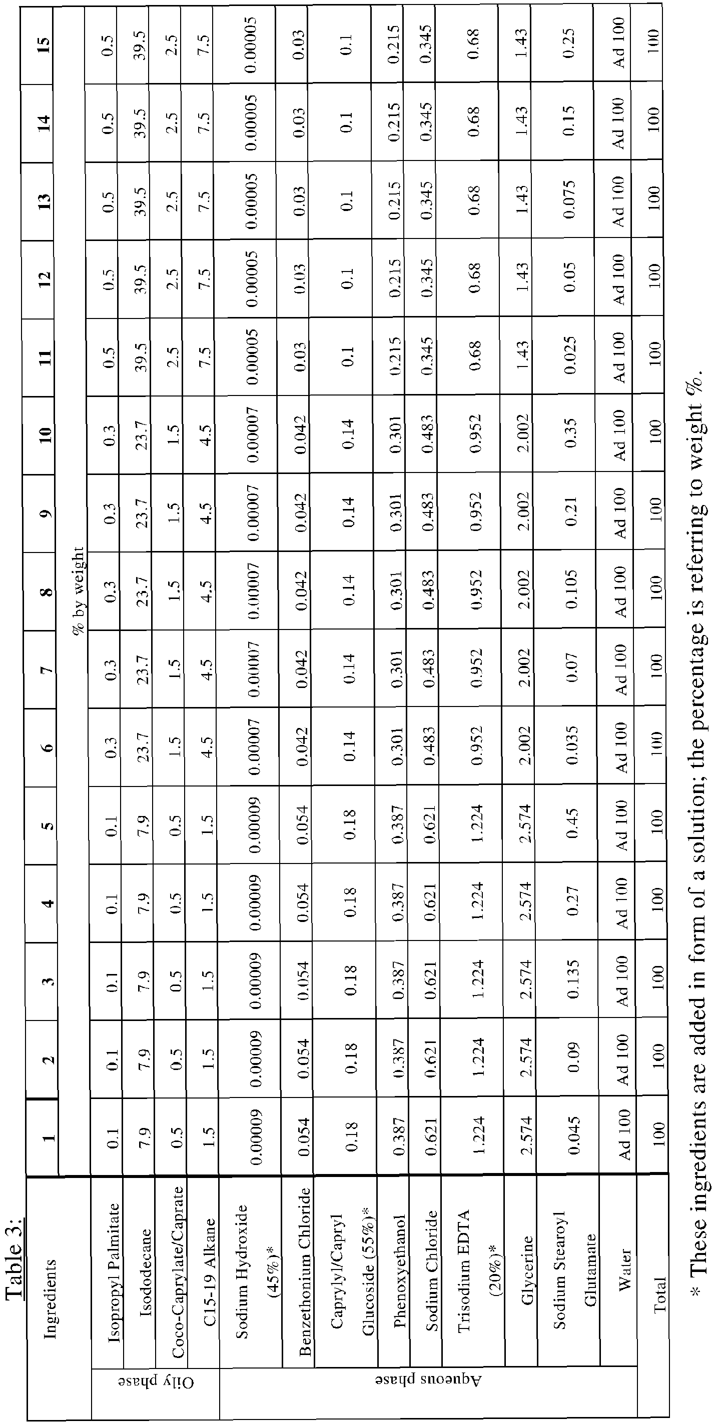

Sample Preparation: The same as indicated for table 1

**Table 4:**

| Ingredients | | % by weight |
|---|---|---|
| Oil Phase | Isopropyl Palmitate | 0.3 |
| | Isododecane | 23.7 |
| | Coco-Caprylate/Caprate | 1.5 |
| | C15-19 Alkane | 4.5 |
| Aqueous Phase | Sodium Hydroxide (45%)* | 0.00007 |
| | Benzethonium Chloride | 0.042 |
| | Caprylyl/Capryl Glucoside (55%)* | 0.14 |
| | Phenoxyethanol | 0.301 |
| | Sodium Chloride | 0.483 |
| | Trisodium EDTA (20%)* | 0.952 |
| | Glycerine | 2.002 |
| | Sodium Stearoyl Glutamate | 0.07 |
| | Water | Ad 100 |
| Total | | 100 |

| | | |
|---|---|---|
| ** These ingredients are added in form of a solution; the percentage is referring to weight %. | | |

### Process Conditions:

| | 1 | 2 | 3 | 4 | 5 | 6 | 7 | 8 |
|---|---|---|---|---|---|---|---|---|
| Homogenization: Speed | 8KRPM | 8KRPM | 8KRPM | 8KRPM | 8KRPM | 3KRPM | 4KRPM | 6KRPM |
| Homogenization: Duration | 20s | 45s | 90s | 180s | 360s | 45s | 45s | 45s |

### Sample Preparation:

1) Weigh the emulsifier according to the amounts shown in the table above and dissolve in water.
2) Use the emulsifier containing water to prepare the aqueous phase as shown in the table above.
3) Prepare the oily phase according to the table above.
4) Mix the oily phase and aqueous phase in a beaker.
5) Homogenize the mixture as shown in the process conditions.
6) Transfer the compositions into sample bottles.

For homogenization an EUROSTAR 20 from the company IKA may be used.

### Effect examples

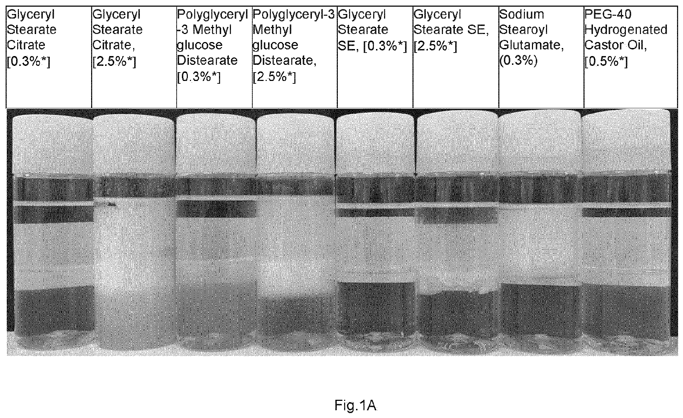

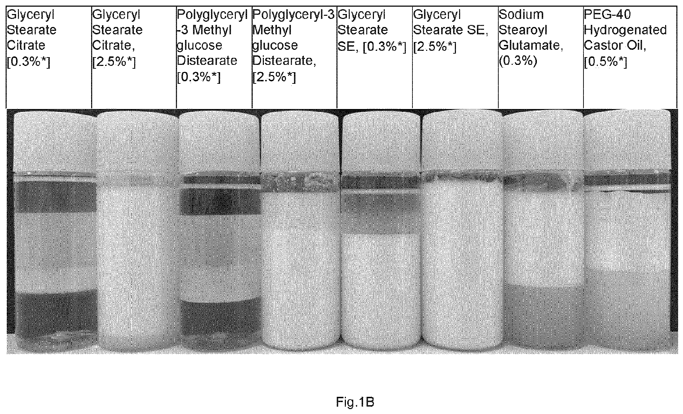

In order to solve the problem to provide a bi-phase make-up remover having a milky upper phase and a clear bottom phase, a selection of promising emulsifiers were evaluated. Compositions were produced (examples shown in table 1) and evaluated (Fig.1A and Fig.1B).

It became apparent that the emulsifier Sodium Stearoyl Glutamate performed best. Sodium Stearoyl Glutamate was used for the following experiments.

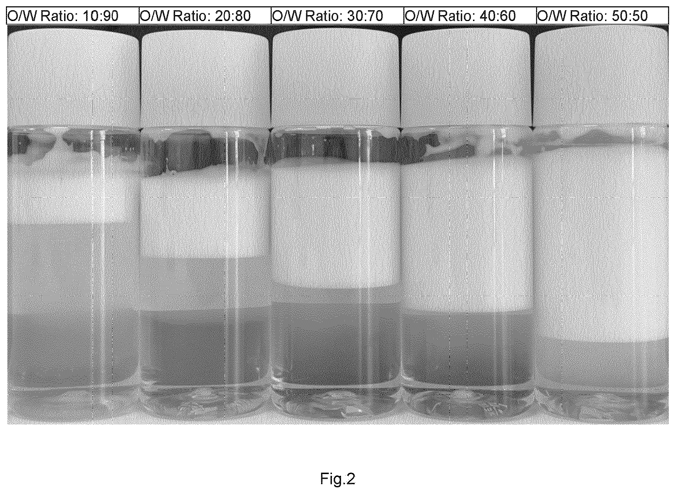

In a second step, the weight ratio of the oily phase to the aqueous phase was analyzed. Again, compositions were produced (examples shown in table 2) and evaluated (Fig.2). Furthermore, the amount of Sodium Stearoyl Glutamate was kept constantly at a concentration of 0.3 % by weight referring to the aqueous phase.

The pictures are showing the example compositions after having been shaken for six to ten seconds and having been allowed to rest for 12 hours. The lower phase is not completely clear after this time of rest, but after a longer time of rest the lower phase turns completely clear.

The results show that the weight ratios of the oily phase to the aqueous phase in the range of 10 : 90 to 50:50 lead to satisfying results.

In a further step, different concentrations of the emulsifier Sodium Stearoyl Glutamate were evaluated in compositions having different weight ratios of the oily phase to the aqueous phase. The respective compositions are shown in table 3 and photos of the respective samples are shown in Fig.3A, Fig.3B and Fig.3C.

Again, the example compositions were shaken for six to ten seconds and then allowed to rest for 12 hours. The lower phase is not completely clear after this time of rest, but after a longer time of rest the lower phase turns completely clear.

The pictures show that all concentrations of the emulsifier Sodium Stearoyl Glutamate and all weight ratios of the oily phase to the aqueous phase tested resulted in satisfying results.

In a fourth step, the conditions of preparation were evaluated. The homogenization was conducted for different time intervals and at different speeds. Also in this case, the example compositions (table 4) were shaken for six to ten seconds and the allowed to rest for 12 hours. The lower phase is not completely clear after this time of rest, but after a longer time of rest the lower phase turns completely clear.

It became apparent that all conditions tested resulted in compositions according to the invention, namely a bi-phase composition with a milky upper phase and a transparent bottom phase.

## Claims

1. A make-up remover composition, especially an eye make-up remover composition, having two visually distinct phases at rest and being converted to a homogenous emulsion upon shaking, containing
- the emulsifier sodium stearoyl glutamate,
- Benzethonium Chloride, and
- an oily phase comprising a least one iso-alkane, at least one alkane and at least one ester oil.

2. Composition according to claim 1 **characterized in that** the composition comprises an upper phase, which is a milky one, and a bottom phase, which is a transparent one.

3. Composition according to claim 1 or 2 **characterized in that** the upper phase is an emulsified oily phase and that the bottom phase is an aqueous phase.

4. Composition according to any one of the preceding claims **characterized in that** the weight ratio of the oily phase to the aqueous phase is from 10 : 90 to 50 : 50.

5. Composition according to any one of the preceding claims **characterized in that** the emulsifier sodium stearoyl glutamate is contained in an amount of 0.05 to 0.5 % by weight, in relation to the active content and in relation to the total weight of the composition.

6. Composition according to any of the preceding claims **characterized in that** the at least one iso-alkane, chosen from iso-alkanes having in total 12 to 40 carbon atoms, preferably 12 to 16 carbon atoms, more preferably isohexadecane and/or isododecane, is contained.

7. Composition according to any one of the preceding claims **characterized in that** the at least one iso-alkane is contained in a total amount of 1.0 to 90 % by weight, preferably 5.0 to 40 % by weight, in relation to the total weight of the composition.

8. Composition according to any one of the preceding claims **characterized in that** the at least one alkane is chosen from unbranched hydrocarbons having 12 to 40 carbon atoms, preferably 15 to 19 carbon atoms, more preferably a blend of hydrocarbons having 15 to 19 carbon atoms.

9. Composition according to any one of the preceding claims **characterized in that** the at least one alkane is contained in a total amount of 1.0 to 15 % by weight, preferably 1.2 to 7.5 % by weight, in relation to the total weight of the composition.

10. Composition according to any one of the preceding claims **characterized in that** the at least one ester oil is an ester of saturated and/or unsaturated, branched and/or unbranched alkane carboxylic acids with a chain length of from 3 to 20 carbon atoms and saturated and/or unsaturated, branched and/or unbranched alcohols with a chain length of from 1 to 20 carbon atoms, and/or an ester of aromatic carboxylic acids and saturated and/or unsaturated, branched and/or unbranched alcohols with a chain length of from 3 to 20 carbon atoms, with the proviso that said esters are fluid oils, preferably the at least one ester oil is chosen from isopropyl myristate, isopropyl palmitate, coco caprylate/caprate, and/or C12-15 -alkyl benzoate.

11. Composition according to any one of the preceding claims **characterized in that** the at least one ester oil is contained in a total amount of 0.05 to 10 % by weight, preferably 0.1 to 3.0 % by weight, in relation to the total weight of the composition.

12. Composition according to any one of the preceding claims **characterized in that** Benzethonium Chloride, is contained in an amount of 0.01 to 0.1 % by weight, preferably 0.03 to 0.06 % by weight, in relation to the total weight of the composition.

13. Composition according to any one of the preceding claims **characterized in that** additionally at least one nonionic surfactant, preferably at least one alkyl poly glucoside is contained.

14. Composition according to claim 13 **characterized in that** the at least one alkyl poly glucoside is caprylyl/capryl glucoside.

15. Composition according to claim 13 or 14 **characterized in that** the at least one alkyl poly glucoside is contained in an amount of 0.05 to 0.5 % by weight, preferably 0.1 to 0.3 % by weight, in relation to the active content and in relation to the total weight of the composition.

16. Composition according to any one of the preceding claims **characterized in that** additionally at least one electrolyte is contained, preferably sodium chloride, whereby it is preferred if the at least one electrolyte is contained in an amount of 0.1 to 2 % by weight, preferably 0.2 to 0.7 % by weight, in relation to the total weight of the composition.

17. Composition according to any one of the preceding claims **characterized in that** the composition is free from silicone components.

## Patentansprüche

1. Make-up-Entferner-Zusammensetzung, insbesondere Augen-Make-up-Entferner-Zusammensetzung, die in Ruhe zwei visuell getrennte Phasen aufweist und die nach Schütteln in eine homogene Emulsion umgewandelt wird, enthaltend
- den Emulgator Natriumstearoylglutamat,
- Benzethoniumchlorid, und
- eine ölige Phase, die mindestens ein Isoalkan, mindestens ein Alkan und mindestens ein Esteröl umfasst.

2. Zusammensetzung nach Anspruch 1, **dadurch gekennzeichnet, dass** die Zusammensetzung eine obere Phase, die eine milchige ist, und eine untere Phase, die eine transparente ist, umfasst.

3. Zusammensetzung nach Anspruch 1 oder 2, **dadurch gekennzeichnet, dass** die obere Phase eine emulgierte ölige Phase ist und dass die untere Phase eine wässrige Phase ist.

4. Zusammensetzung nach einem der vorhergehenden Ansprüche, **dadurch gekennzeichnet, dass** das Gewichtsverhältnis der öligen Phase zu der wässrigen Phase sich beläuft auf 10 : 90 bis 50 : 50.

5. Zusammensetzung nach einem der vorhergehenden Ansprüche, **dadurch gekennzeichnet, dass** der Emulgator Natriumstearoylglutamat in einer Menge von 0,05 bis 0,5 Gew.-%, bezogen auf den Aktivgehalt und bezogen auf das Gesamtgewicht der Zusammensetzung, enthalten ist.

6. Zusammensetzung nach einem der vorhergehenden Ansprüche, **dadurch gekennzeichnet, dass** das mindestens eine Isoalkan, ausgewählt aus Isoalkanen mit insgesamt 12 bis 40 Kohlenstoffatomen, bevorzugt 12 bis 16 Kohlenstoffatomen, besonders bevorzugt Isohexadecan und/oder Isododecan, enthalten ist.

7. Zusammensetzung nach einem der vorhergehenden Ansprüche, **dadurch gekennzeichnet, dass** das mindestens eine Isoalkan in einer Gesamtmenge von 1,0 bis 90 Gew.- %, vorzugsweise 5,0 bis 40 Gew.-%, bezogen auf das Gesamtgewicht der Zusammensetzung, enthalten ist.

8. Zusammensetzung nach einem der vorhergehenden Ansprüche, **dadurch gekennzeichnet, dass** das mindestens eine Alkan ausgewählt ist aus unverzweigten Kohlenwasserstoffen mit 12 bis 40 Kohlenstoffatomen, bevorzugt 15 bis 19 Kohlenstoffatomen, besonders bevorzugt einem Gemisch von Kohlenwasserstoffen mit 15 bis 19 Kohlenstoffatomen.

9. Zusammensetzung nach einem der vorhergehenden Ansprüche, **dadurch gekennzeichnet, dass** das mindestens eine Alkan in einer Gesamtmenge von 1,0 bis 15 Gew.-%, vorzugsweise 1,2 bis 7,5 Gew.-%, bezogen auf das Gesamtgewicht der Zusammensetzung, enthalten ist.

10. Zusammensetzung nach einem der vorhergehenden Ansprüche, **dadurch gekennzeichnet, dass** das mindestens eine Esteröl ein Ester gesättigter und/oder ungesättigter, verzweigter und/oder unverzweigter Alkancarbonsäuren mit einer Kettenlänge von 3 bis 20 Kohlenstoffatomen und gesättigter und/oder ungesättigter, verzweigter und/oder unverzweigter Alkohole mit einer Kettenlänge von 1 bis 20 Kohlenstoffatomen und/oder ein Ester aromatischer Carbonsäuren und gesättigter und/oder ungesättigter, verzweigter und/oder unverzweigter Alkohole mit einer Kettenlänge von 3 bis 20 Kohlenstoffatomen ist, mit der Maßgabe, dass es sich bei diesen Estern um fluide Öle handelt, wobei bevorzugt das mindestens eine Esteröl ausgewählt ist aus Isopropylmyristat, Isopropylpalmitat, Cococaprylat/caprat und/oder C12-15-Alkylbenzoat.

11. Zusammensetzung nach einem der vorhergehenden Ansprüche, **dadurch gekennzeichnet, dass** das mindestens eine Esteröl in einer Gesamtmenge von 0,05 bis 10 Gew.- %, vorzugsweise 0,1 bis 3,0 Gew.-%, bezogen auf das Gesamtgewicht der Zusammensetzung, enthalten ist.

12. Zusammensetzung nach einem der vorhergehenden Ansprüche, **dadurch gekennzeichnet, dass** Benzethoniumchlorid in einer Menge von 0,01 bis 0,1 Gew.- %, vorzugsweise 0,03 bis 0,06 Gew.-%, bezogen auf das Gesamtgewicht der Zusammensetzung, enthalten ist.

13. Zusammensetzung nach einem der vorhergehenden Ansprüche, **dadurch gekennzeichnet, dass** zusätzlich mindestens ein nichtionisches Tensid, vorzugsweise mindestens ein Alkylpolyglucosid enthalten ist.

14. Zusammensetzung nach Anspruch 13, **dadurch gekennzeichnet, dass** das mindestens eine Alkylpolyglucosid Caprylyl/Caprylglucosid ist.

15. Zusammensetzung nach Anspruch 13 oder 14, **dadurch gekennzeichnet, dass** das mindestens eine Alkylpolyglucosid in einer Menge von 0,05 bis 0,5 Gew.- %, vorzugsweise 0,1 bis 0,3 Gew.-%, bezogen auf den Aktivgehalt und bezogen auf das Gesamtgewicht der Zusammensetzung, enthalten ist.

16. Zusammensetzung nach einem der vorhergehenden Ansprüche, **dadurch gekennzeichnet, dass** zusätzlich mindestens ein Elektrolyt enthalten ist, vorzugsweise Natriumchlorid, wobei es bevorzugt ist, wenn der mindestens eine Elektrolyt in einer Menge von 0,1 bis 2 Gew.-%, vorzugsweise 0,2 bis 0,7 Gew.-%, bezogen auf das Gesamtgewicht der Zusammensetzung, enthalten ist.

17. Zusammensetzung nach einem der vorhergehenden Ansprüche, **dadurch gekennzeichnet, dass** die Zusammensetzung frei von Silikonkomponenten ist.

## Revendications

1. Composition démaquillante, notamment composition démaquillante pour les yeux, présentant deux phases visuellement distinctes au repos et qui est convertie en une émulsion homogène après agitation, contenant
- l'émulsifiant stéaroylglutamate de sodium,
- du chlorure de benzéthonium, et
- une phase huileuse comprenant au moins un iso-alcane, au moins un alcane et au moins une huile d'ester.

2. Composition selon la revendication 1, **caractérisée en ce que** la composition comprend une phase supérieure, qui est une phase laiteuse, et une phase inférieure, qui est une phase transparente.

3. Composition selon la revendication 1 ou 2, **caractérisée en ce que** la phase supérieure est une phase huileuse émulsifiée et **en ce que** la phase inférieure est une phase aqueuse.

4. Composition selon l'une quelconque des revendications précédentes, **caractérisée en ce que** le rapport pondéral de la phase huileuse sur la phase aqueuse est de 10 : 90 à 50 : 50.

5. Composition selon l'une quelconque des revendications précédentes, **caractérisée en ce que** l'émulsifiant stéaroylglutamate de sodium est contenu en une quantité de 0,05 à 0,5 % en poids, par rapport à la teneur en substance active et par rapport au poids total de la composition.

6. Composition selon l'une quelconque des revendications précédentes, **caractérisée en ce que** l'au moins un iso-alcane, choisi parmi les iso-alcanes ayant au total 12 à 40 atomes de carbone, de préférence 12 à 16 atomes de carbone, plus préférentiellement l'isohexadécane et/ou l'isododécane, est contenu.

7. Composition selon l'une quelconque des revendications précédentes, **caractérisée en ce que** l'au moins un iso-alcane est contenu en une quantité totale de 1,0 à 90 % en poids, de préférence de 5,0 à 40 % en poids, par rapport au poids total de la composition.

8. Composition selon l'une quelconque des revendications précédentes, **caractérisée en ce que** l'au moins un alcane est choisi parmi les hydrocarbures non ramifiés ayant 12 à 40 atomes de carbone, de préférence 15 à 19 atomes de carbone, plus préférentiellement un mélange d'hydrocarbures ayant 15 à 19 atomes de carbone.

9. Composition selon l'une quelconque des revendications précédentes, **caractérisée en ce que** l'au moins un alcane est contenu en une quantité totale de 1,0 à 15 % en poids, de préférence de 1,2 à 7,5 % en poids, par rapport au poids total de la composition.

10. Composition selon l'une quelconque des revendications précédentes **caractérisée en ce que** l'au moins une huile d'ester est un ester d'acides alcane carboxyliques saturés et/ou insaturés, ramifiés et/ou non ramifiés ayant une longueur de chaîne de 3 à 20 atomes de carbone et d'alcools saturés et/ou insaturés, ramifiés et/ou non ramifiés ayant une longueur de chaîne de 1 à 20 atomes de carbone, et/ou un ester d'acides carboxyliques aromatiques et d'alcools saturés et/ou insaturés, ramifiés et/ou non ramifiés présentant une longueur de chaîne de 3 à 20 atomes de carbone sous réserve que lesdits esters soient des huiles fluides, de préférence l'au moins une huile d'ester est choisie parmi le myristate d'isopropyle, le palmitate d'isopropyle, le caprylate/caprate de coco, et/ou un benzoate d'alkyle en C12-15.

11. Composition selon l'une quelconque des revendications précédentes, **caractérisée en ce que** l'au moins une huile d'ester est contenue en une quantité totale de 0,05 à 10 % en poids, de préférence de 0,1 à 3,0 % en poids, par rapport au poids total de la composition.

12. Composition selon l'une quelconque des revendications précédentes, **caractérisée en ce que** le chlorure de benzéthonium est contenu en une quantité totale de 0,01 à 0,1 % en poids, de préférence de 0,03 à 0,06 % en poids, par rapport au poids total de la composition.

13. Composition selon l'une quelconque des revendications précédentes, **caractérisée en ce que** de plus au moins un tensioactif non ionique, de préférence au moins un alkylpolyglucoside est contenu.

14. Composition selon la revendication 13 **caractérisée en ce que** l'au moins un alkylpolyglucoside est un caprylyl/capryl-glucoside.

15. Composition selon la revendication 13 ou 14, **caractérisée en ce que** l'au moins un alkylpolyglucoside est contenu en une quantité de 0,05 à 0,5 % en poids, de préférence de 0,1 à 0,3 % en poids, par rapport à la teneur en substance active et par rapport au poids total de la composition.

16. Composition selon l'une quelconque des revendications précédentes, **caractérisée en ce que** de plus au moins un électrolyte est contenu, de préférence du chlorure de sodium, moyennant quoi il est préféré que l'au moins un électrolyte soit contenu en une quantité de 0,1 à 2 % en poids, de préférence de 0,2 à 0,7 % en poids, par rapport au poids total de la composition.

17. Composition selon l'une quelconque des revendications précédentes, **caractérisée en ce que** la composition est exempte de composants silicone.
